# EUROPEAN PATENT APPLICATION

(11) **EP 3 904 370 A1**
(43) Date of publication of application: **03.11.2021**
(21) Application number: 20172070.3
(22) Date of filing: 29.04.2020
(51) Int. Cl.: C07K 14/195, C07K 14/245, C07K 14/435, C12N 1/20, C12P 19/12

(54) **A NEW METHOD FOR THE PRODUCTION OF TREHALOSE FROM GASEOUS SUBSTRATES**

(71) Applicant: Technische Universität München, 80333 München (DE)
(72) Inventor: Löwe, Hannes, 80805 München (DE); Pflüger-Grau, Katharina, 81927 München (DE); Kremling, Andreas, 85567 Grafing (DE)
(74) Representative: Engelhard, Markus

(57) **Abstract**

The present invention relates to a genetically modified organism, wherein said organism is selected from organisms having a chemolithotrophic and/or methylotrophic metabolism, wherein said organism expresses a sugar efflux transporter. The present invention also relates to a method of producing trehalose and/or glucose. The present invention further relates to a use of a genetically modified organism for producing trehalose and/or glucose.

## Description

### FIELD OF THE INVENTION

The present invention relates to a genetically modified organism, wherein said organism is selected from organisms having a chemolithotrophic and/or methylotrophic metabolism, wherein said organism expresses a sugar efflux transporter. The present invention also relates to a method of producing trehalose and/or glucose. The present invention further relates to a use of a genetically modified organism for producing trehalose and/or glucose.

### BACKGROUND OF THE INVENTION

The massive release of CO₂ caused by burning fossil fuels and the associated global warming of the earth's atmosphere and oceans is probably one of the biggest challenges mankind ever faced. Besides the development of renewable energy, scientist and engineers are working to replace fossil resources by natural substrates, like sugar or plant oil, for the production of fuels and chemicals. In order to achieve this, organic compounds derived from plants that fix CO₂, have to be used as a starting material to make the process renewable. For the production of relevant amounts of fuels and chemicals, a big area has to be dedicated to this bioeconomy.

About 8-9 % of the agricultural area in the EU is used for non-food production. A major part of the agricultural area used for non-food production accounts for oil production that is mainly used for fuels and chemicals. With a growing world population and a big gap to close between a fossil and a renewable economy, it is clear that food and fuel/chemical production from crops are in competition for the agricultural area. Therefore, resource-efficient means for providing feedstock, such as energy in the form of sugar, without using large areas of agricultural land, are needed. The present invention aims at providing a microbiological process that can produce the sugar trehalose and/or glucose, in a resource-efficient way such as from CO₂ and hydrogen gas that can be obtained from alternative source like biomass gasification, electrolysis or reformation of methane. Furthermore, the present invention aims at producing carbohydrates as substrates for industrial biotechnology with higher areal efficiencies than crops.

Trehalose is a natural occurring carbohydrate built of two glucose molecules. It is produced by many organisms in situations of stress, especially as a result of salt or desiccation stress. Because of its protective nature, it is not only used by many microorganisms, but also in the food and cosmetics industries for different purposes, as well as in medical formulations. It is also less cariogenic than other sugars and mitigates insulin resistance making it interesting for sugar replacement in food for diabetes patients.

At the moment, trehalose is produced mainly enzymatically from starch, but the direct production from maltose by the enzyme trehalose synthase is a matter of ongoing research [1]. The current enzymatic processes all rely on purified enzymes and on carbon sources from crop plants that have to be planted, harvested and refined.

There have been attempts to genetically modify organisms for producing carbohydrates such as trehalose. US 9284519 B2 and US 6800474 B1 relate to genetically modified organisms with increased carbohydrate production by overexpression of trehalose synthesis genes. Furthermore, organisms have been genetically modified to expand the substrate spectrum thereof [2]. EP2719757A1 presents a new method for producing sugars from CO₂ with genetically modified cyanobacteria by the expression of suitable transport proteins. US 7892789 B2 relates to providing a trehalose transporter gene to a Xenopus oocyte to increase trehalose permeability of the cell membrane. It has been observed that trehalose may accumulate in bacteria and that culture conditions have an effect on trehalose yield [3]. Furthermore, *Cupriavidus necator* H16 has been studied with regard to utilization of different substrates [4]. However, there remains the need for an efficient means for producing sugars such as trehalose and/or glucose in microorganisms.

Sugar production from CO₂ has mainly been investigated in cyanobacteria that rely on light as the main energy source, which only scales up by large surface areas. This, among other reasons, has prevented the commercialization of processes based on cyanobacteria on a large scale for low-value products such as sugars. An alternative pathway to use CO₂ is synthesis gas fermentation, e.g. with acetogens, involving a mixture of H₂, CO₂ and CO that has gained a lot of attention in the recent years since it provides a relatively high yield of short organic acids and alcohols such as acetic acid and ethanol, the substrates are relatively low priced, and it is able to fix CO₂. *Cupriavidus necator* has been used for producing sucrose from CO2 under lithotrophic conditions [5]. However, many metabolites, such as sugars, cannot be produced under anaerobic conditions as the processes are not thermodynamically feasible without massive by-production of acetic acid. Thus, an aim of the present invention is to provide efficient means for producing sugars from CO₂ without the areal demand of crop or microalgae culture by using hydrogen based gas mixtures derived from waste biomass gasification or electrolysis. The present invention further aims at providing an efficient process that can be performed with whole cells, i.e. without the need for enzyme purification, and that uses easily available substrates such as gaseous substrates, e.g. a mixture of hydrogen gas, carbon dioxide and oxygen. The present invention also aims at providing an organism with increased secretion of sugars such as trehalose and/or glucose.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a genetically modified organism according to the appended claims, wherein said organism is selected from organisms having a chemolithotrophic and/or methylotrophic metabolism, wherein said organism expresses a sugar efflux transporter, preferably a sugar uniporter or a cation/sugar antiporter, optionally further overexpresses one or more trehalose synthesis gene(s).

In one embodiment, said expression, optionally overexpression, may comprise an expression of an exogenous gene of said sugar efflux transporter, optionally of said one or more trehalose synthesis gene(s), in said organism, and/or may comprise an increased expression of an endogenous gene of said sugar efflux transporter, optionally of said one or more trehalose synthesis gene(s), in said organism.

In another embodiment, said organism may be a chemolithotrophic organism, preferably selected from but not limited to hydrogen oxidizing bacteria, autotrophic phosphite oxidizing bacteria, autotrophic nitrate/nitrite or sulfate respiring microorganisms, and genetically modified organisms having obtained chemolithotrophic characteristics by genetic modification, more preferably *Cupriavidus necator.*

In yet another embodiment, said organism may be a methylotrophic organism, preferably any organism selected from but not limited to Cupriavidus sp., such as *Cupriavidus necator H16,* Bacillus sp., such as *Bacillus methanolicus,* Methylobacterium sp., such as *Methylobacterium extorquens,* Hydrogenophaga sp., such as *Hydrogenophaga pseudoflava,* Rhodococcus sp., such as *Rhodococcus opacus,* Ralstonia sp., Pichia sp., such as *Pichia pastoris,* Komagatella sp., and Paracoccus sp., such as *Paracoccus denitrificans,* and genetically modified organisms having obtained methylotrophic characteristics by genetic modification.

In a further embodiment, said sugar efflux transporter may be any of a sugar uniporter, a H+ or Na⁺-antiporter, and/or an ATP-dependent efflux transport protein, preferably is a sugar uniporter or H⁺/sugar-antiporter selected from but not limited to the sugar efflux transporter A (SetA) from *Escherichia coli* (SEQ ID NO. 1), the glucose facilitator Glf from *Zymomonas mobilis* (SEQ ID NO. 2), the transport protein YdeA from *E. coli* (SEQ ID NO. 3), a protein of the SWEET transport protein family, the Tret1-1 trehalose facilitator from *Drosophila melanogaster* (SEQ ID NO. 4), a protein from the (GLUT)/SLC2A transport protein family, and homologues and variants thereof, more preferably is SetA.

In another embodiment, SetA may be derived from *E. coli,* preferably SetA having SEQ ID NO. 1, and/or may be a SetA homologue or variant.

In yet another embodiment, said one or more trehalose synthesis gene(s) may be selected from phosphoglucosemutase, UTP-glucose-1-phosphate uridylyltransferase, trehalose phosphate synthetase, trehalose-phosphate phosphatase, maltooligosyl trehalose synthase, and maltooligosyl trehalose hydrolase.

In another embodiment, said organism may be transformed with pSEVA228-*setA* having SEQ ID NO. 5.

In a further embodiment, said organism may be *Cupriavidus necator* Cn_H16_*setA* deposited at the Deutsche Sammlung von Mikroorganismen und Zellkulturen GmbH having accession number DSM 33415.

In a further aspect, the present invention relates to a method of producing trehalose and/or glucose according to the appended claims, wherein said method comprises the following steps:
i) providing a genetically modified organism as defined in any of the embodiments above and/or a genetically modified organism expressing a sugar efflux transporter, preferably SetA, optionally further overexpressing one or more trehalose synthesis gene(s);
ii) culturing the genetically modified organism of step i) in a cell culture, said culturing comprising supplying an inorganic electron source, preferably H₂ in a gaseous mixture comprising H₂, O₂, and CO₂, and/or phosphite, and/or supplying any of nitrate as electron acceptor, carbonate, or an organic carbon source, such as fructose, succinic acid, acetic acid, glycerol, and mixtures thereof; and
iii) obtaining trehalose and/or glucose from said cell culture.

In an embodiment, in step iii), said trehalose and/or glucose may be obtained from a supernatant of said cell culture.

In another embodiment, in step ii), said gaseous mixture may comprise 20-99% (v/v) H₂, 0.1-20% (v/v) O₂, and 0.1-20% (v/v) CO₂.

In yet another embodiment, in step ii), said H₂, O₂, and CO₂ may be supplied in a ratio (v/v) of 6:1:1, respectively, to said cell culture.

In a further embodiment, in step iii), said obtaining of glucose may further comprise enzymatic cleavage of trehalose by trehalase to obtain glucose.

In another embodiment, said method may be a method of producing isotope-labelled trehalose and/or isotope-labelled glucose, said method comprising
in step ii), supplying an inorganic electron source, preferably H₂ in a gaseous mixture comprising H₂, isotope-labelled CO₂, and O₂, and/or phosphite, and/or supplying nitrate as an electron acceptor, and/or supplying any of isotope-labelled carbonate, fructose, succinic acid, acetic acid, glycerol, and mixtures thereof, and,
in step iii), obtaining isotope-labelled trehalose and/or isotope-labelled glucose, preferably isotope-labelled glucose.

In a further aspect, the present invention relates to a use of a genetically modified organism according to the appended claims and as defined in any of the embodiments above, for producing trehalose and/or glucose, optionally isotope-labelled trehalose and/or isotope labelled glucose.

### DETAILED DESCRIPTION

An advantage of the present invention is that the use of agricultural land for providing feedstock is avoided by an organism and a method of the present invention, e.g. means for an area-efficient production of sugars are provided. A further advantage of an organism and a method of the present invention is that the greenhouse gas CO₂ can be bound in the form of trehalose. A method of the present invention is further advantageous in that it is more sustainable than common methods of producing trehalose, since it utilizes residual biomass, biogas, and/or hydrogen from electrolysis, and thus needs less agricultural land and water. A further advantage of a method of the present invention is that it is more cost-efficient than existing methods for trehalose production. The present invention is further advantageous in that chemolithotrophic organisms and methylotrophic organisms, particularly hydrogen oxidizing bacteria, can directly breathe H₂/O₂ mixtures and thus produce enough ATP to produce energy intensive metabolites such as trehalose. A further advantage of the present invention is that isotopically labelled trehalose and/or glucose can easily and efficiently be produced from ¹³CO₂. The present invention is also advantageous in that a sugar such as trehalose can be obtained directly from a cell culture, since the organisms used in the present invention, for example genetically modified organisms, are able to metabolize a gaseous substrate.

The present invention provides means for obtaining sugars, particularly trehalose and/or glucose, by overexpressing a sugar efflux protein, preferably SetA, in a microorganism. The sugars produced by said microorganism are secreted into the cell culture medium by means of said sugar efflux protein.

The term "genetically modified organism" relates to a cell comprising a genetic modification. In one embodiment, a genetically modified organism comprises a genetic modification with respect to a sugar efflux transporter, preferably an expression of an exogenous sugar efflux transporter and/or an enhanced expression of an endogenous sugar efflux transporter. In one embodiment, a genetically modified organism is any of a hydrogen oxidizing bacterium, an autotrophic phosphite oxidizing bacterium, an autotrophic nitrate/nitrite or sulfate respiring microorganism, Cupriavidus sp., such as *Cupriavidus necator H16,* Bacillus sp., such as *Bacillus methanolicus,* Methylobacterium sp., such as *Methylobacterium extorquens,* Hydrogenophaga sp., such as *Hydrogenophaga pseudoflava,* Rhodococcus sp., such as *Rhodococcus opacus,* Ralstonia sp., Pichia sp., such as *Pichia pastoris,* Komagatella sp., Paracoccus sp., such as *Paracoccus denitrificans, Escherichia coli, Pseudomonas putida, Halomonas elongata, Synechococcus elongatus,* and a cyanobacterium, preferably is *Cupriavidus necator.* In one embodiment, an organism of the present invention is an organism with a chemolithotrophic and/or methylotrophic metabolism. In one embodiment, an organism of the present invention may have chemolithotrophic and/or methylotrophic properties depending on the substrate provided. In one embodiment, a genetically modified organism of the present invention is preferably *C. necator,* such as *C. necator H16.* In one embodiment, a genetically modified organism of the present invention is capable of growing using a gaseous mixture comprising CO₂, O₂, and H₂ as a substrate, optionally is a hydrogen oxidizing bacterium.

In one embodiment, said genetically modified organism is *Cupriavidus necator* Cn_H16_*setA* deposited on January 22, 2020 by the Technical University of Munich at the Deutsche Sammlung von Mikroorganismen und Zellkulturen GmbH having accession number DSM 33415.

In one embodiment, said genetically modified organism is further modified by heterologous expression of a CO dehydrogenase. In one embodiment, said genetically modified organism expressing a CO dehydrogenase is able to metabolize CO as a substrate. In one embodiment, said genetically modified organism has been further genetically modified by deleting a membrane bound hydrogenase so that said further genetically modified organism uses electrons obtained from H₂ for biosynthesis of sugars.

The term "hydrogen oxidizing bacterium", as used herein, relates to a bacterium that is capable of growing having a gaseous mixture comprising CO₂, O₂, and H₂ as a substrate. *Cupriavidus necator* H16 is an exemplary hydrogen oxidizing bacterium, which is a gram-negative bacterium that is a natural producer of polyhydroxybutyrate (PHB). It has been shown that *Cupriavidus necator* is able to use synthesis gas and/or CO as an energy source and that it is able to grow at elevated CO concentrations. In one embodiment, *Cupriavidus necator* H16 is a preferred microorganism in this advantageous in that it has fast growth rates and is not oxygen-sensitive. In one embodiment, *Cupriavidus necator* H16 comprises three systems for producing trehalose, namely an *otsAB* operon having homology to the respective genes in *E. coli K12,* a *treYZ* operon, and a trehalose synthase *treS* with homology to the respective genes in *Pseudomonas putida* KT2440. In one embodiment, the terms "hydrogen oxidizing bacterium" and "Knallgas bacterium" are used interchangeably. In one embodiment, an organism of the present invention does not have the capability of a chemolithotrophic and/or methylotrophic metabolism, until being genetically modified. In one embodiment, an organism obtains genes necessary for being capable of a chemolithotrophic and/or methylotrophic metabolism by genetic modification, and thereby becomes a genetically modified organism of the present invention.

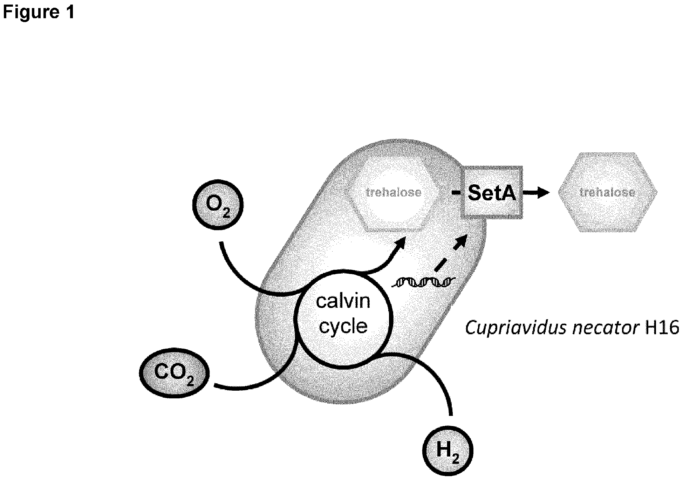

The present inventors genetically modified hydrogen oxidizing bacterium *Cupriavidus necator* H16, formerly known as *Ralstonia eutropha* H16, in order to direct its metabolism towards the secretion of trehalose. This was achieved by overexpressing a sugar-efflux transporter, for example SetA from *E. coli* BL21 (DE3) in said hydrogen oxidizing bacterium, optionally subjecting it to elevated salt concentrations. A schematic representation of the cellular process is given in Figure 1. In one embodiment, a genetically modified organism of the present invention further comprises an exogenous carbon monoxide dehydrogenase.

The term "chemolithotrophic", as used herein, relates to organisms using an inorganic substrate for biosynthesis, e.g. carbon dioxide fixation, or energy conservation via aerobic or anaerobic respiration. In chemolithotrophs, the substrates (electron donors) are oxidized in the cell, and the electrons are channeled into respiratory chains, resulting in the production of ATP. In one embodiment, a chemolithotrophic organism is selected from hydrogen oxidizing bacteria (Knallgas bacteria), autotrophic phosphite oxidizing bacteria, autotrophic nitrate/nitrite or sulfate respiring microorganisms, and genetically modified organisms having obtained chemolithotrophic characteristics by genetic modification, more preferably *Cupriavidus necator.* Knallgas bacteria are bacteria that are able to grow using a gaseous mixture of CO₂, O₂ und H₂ as a substrate. In one embodiment, a method of the present invention comprises providing a mixture of CO₂, O₂ and H₂ as a substrate, and/or providing a mixture of CO₂, H₂, and nitrate as a substrate, and/or providing a mixture of CO₂, O₂, and phosphite as a substrate, and/or providing a mixture of CO₂, O₂, H₂, CO, and optionally NH₃, as a substrate, preferably providing a mixture of CO₂, O₂ and H₂ as a substrate.

The term "methylotrophic", as used herein, relates to microorganisms that can use C₁ carbon compounds, such as methanol, methane, or formic acid, as the carbon source for their growth. In one embodiment, methylotrophic organisms are also capable of using multi-carbon compounds that contain no carbon-carbon bonds, such as dimethyl ether and dimethylamine, and/or capable of using compounds such as carbon dioxide, as a substrate. In one embodiment, a methylotrophic organism is selected from Cupriavidus sp., such as *Cupriavidus necator H16,* Bacillus sp., such as *Bacillus methanolicus,* Methylobacterium sp., such as *Methylobacterium extorquens,* Hydrogenophaga sp., such as *Hydrogenophaga pseudoflava,* Rhodococcus sp., such as *Rhodococcus opacus,* Ralstonia sp., Pichia sp., such as *Pichia pastoris,* Komagatella sp., and Paracoccus sp., such as *Paracoccus denitrificans,* and genetically modified organisms having obtained methylotrophic characteristics by genetic modification. In one embodiment, an organism may be capable of a methylotrophic and/or chemolithotrophic metabolism.

The term "expresses", as used herein, relates to a genetically modified organism comprising a target gene and producing the protein encoded by said target gene. In one embodiment, the expression of said target gene in said genetically modified organism is increased compared to in an organism that has not been genetically modified. In one embodiment, said expression, optionally overexpression, comprises an expression of an exogenous gene of said target gene in said organism, and/or comprises an increased expression of an endogenous gene of said target gene in said organism. In one embodiment, said target gene is a gene encoding a sugar efflux transporter or encoding a protein involved in trehalose synthesis. In one embodiment, the term "exogenous" relates to a gene which does not naturally occur in an organism, but is artificially introduced into said organism. In one embodiment, the term "endogenous" relates to a gene which naturally occurs in said organism, and which is optionally artificially overexpressed, e.g. by gene duplication and/or insertion of an expression-enhancing promotor. In one embodiment, a genetically modified organism expresses an exogenous sugar efflux transporter and/or expresses an endogenous sugar efflux transporter, optionally overexpresses an endogenous sugar efflux transporter. In one embodiment, the term "overexpression" relates to an increased expression.

The term "transporter" or "transport protein", as used herein, relates to a membrane transport protein involved in the movement of ions, small molecules, and macromolecules, across a biological membrane. The proteins may assist in the movement of substances by facilitated diffusion or active transport, and may provide ATP-dependent or ATP-independent transport. In one embodiment, the term relates to a sugar efflux transporter.

In one embodiment, the transporter is genetically optimized for efficient sugar transportation, namely by directed mutagenesis. In one embodiment, said genetic optimization is performed by error-prone PCR and subsequent screening of the target candidates. In one embodiment, said screening comprises expressing a candidate in a host organism, such as *E. coli,* which is deficient in trehalose transport and in periplasmatic trehalase. Such a host organism can only grow as fast as the trehalose uptake takes place. Accordingly, the growth rate of the host organism indicates the transporter activity of the respective candidate. The candidates having the highest transporter activity can then be used for a genetically modified organism of the present invention.

The term "sugar efflux transporter", as used herein, relates to a transporter that mediates secretion/release of sugar from a cell. In one embodiment, said sugar efflux transporter is a sugar uniporter, a cation/sugar antiporter such as a H⁺ or Na⁺-antiporter, and/or an ATP-dependent efflux transport protein. A sugar "uniporter" is a transport protein that transports a single type of molecule or ion across a cell membrane, either by facilitated diffusion and transport along a diffusion gradient or by an active transport process. A "cation/sugar antiporter", as used herein, relates to an antiporter that transports a sugar, such as trehalose or glucose, and a cation, such as H⁺ or Na⁺, in opposite directions across a membrane. A H⁺ or Na⁺-antiporter, as used herein, relates to a transport protein transporting i) sugar and ii) H⁺ or Na⁺ across a membrane in opposite directions. In one embodiment, the terms "H⁺ or Na⁺-antiporter" and "H⁺/sugar- or Na⁺/sugar-antiporter" are used interchangeably. An ATP-dependent efflux transport protein is a transport protein mediating active transport using ATP. In one embodiment, said sugar efflux transporter is preferably a sugar uniporter or H+/sugar-antiporter. In one embodiment, said sugar efflux transporter is selected from SetA, Glf, YdeA, the SWEET transport protein family, TRET1, and the (GLUT)/SLC2A transport protein family, preferably is SetA. The SWEET transport protein family (2.A.123, transport classification database) is a family of sugar transporters and a member of the TOG superfamily. TRET1 relates to a facilitated trehalose transporter. The (GLUT)/SLC2A transport protein family relates to a family of glucose transporters. In one embodiment, a sugar efflux transporter is a protein having a sequence of any of SEQ ID NO. 1 to 4, and/or is a homologue or variant thereof having at least 80 %, preferably at least 90 %, more preferably at least 95 % sequence identity with any of SEQ ID NO. 1 to 4. In one embodiment, a genetically modified organism of the present invention expresses a sugar efflux transporter which is preferably SetA, and/or a homologue or variant thereof.

In one embodiment, a sugar efflux transporter is not a sugar symporter such as a saccharose/H⁺-symporter, e.g. a sugar efflux transporter is not sucrose permease CscB. In one embodiment, the sugar efflux transporter is not a sucrose efflux transporter. In one embodiment, the sugar efflux transporter does not transport sucrose. In one embodiment, the sugar efflux transporter preferably transports any of trehalose and glucose.

The term "SetA", as used herein, relates to sugar efflux transporter A which is involved in the efflux of sugars. In one embodiment, SetA derives from *Escherichia coli* strain K12 and/or *Escherichia coli* strain BL21. In one embodiment, the term "SetA" refers to SetA, SetA homologues and SetA variants. A "SetA homologue", as used herein, is any homologue of SetA found in any other organism, for example a SetA homologue found in Enterobacteriaceae sp., Streptococcus sp, such as *Streptococcus pneuminae,* Pseudomonas sp., such as Pseudomonas aeruginosa, Achromobacter sp., and Corynebacterium sp. For example, a SetA homologue may relate to a sugar efflux system from E. coli such as *ydeA* which is an arabinose/H⁺-antiporter mediating secretion of arabinose. The term "SetA variant" relates to a biologically active variant of SetA or of a SetA homologue, e.g. a mutated variant, a truncated variant, and a variant obtained by protein engineering and/or genetic modification of SetA or of a SetA homologue, such as optimization by directed mutagenesis. In one embodiment, SetA has a sequence of SEQ ID NO. 1. In one embodiment, a SetA variant has at least 80 %, preferably at least 90 %, more preferably at least 95 % sequence identity with a sequence of SEQ ID NO. 1. In one embodiment, SetA relates to a protein having UniProt accession number A0A140NG97. In one embodiment, the term "homologue" of a sugar efflux transporter relates to any homologue of said sugar efflux transporter found in any other organism than said sugar efflux transporter. In one embodiment, the term "variant" of a sugar efflux transporter relates to any biologically active variant of said sugar efflux transporter, such as a mutated variant, a truncated variant, and a variant obtained by protein engineering and/or genetic modification. In one embodiment, a variant of a sugar efflux transporter has at least 80 %, preferably at least 90 %, more preferably at least 95 % sequence identity with said sugar efflux transporter.

The term "trehalose synthesis gene", as used herein, relates to a gene encoding a protein involved in trehalose synthesis. In one embodiment, a trehalose synthesis gene is any of phosphoglucosemutase, UTP-glucose-i-phosphate uridylyltransferase, trehalose phosphate synthetase, such as of the trehalose-6-phosphate synthase/phosphatase family (otsAB), trehalose-phosphate phosphatase, maltooligosyl trehalose synthase, and maltooligosyl trehalose hydrolase.

The term "genetically modified organism expressing a sugar efflux transporter", as used in a method of the present invention, relates to an organism genetically modified to express a sugar efflux transporter. Said genetically modified organism expressing a sugar efflux transporter used in a method of the present invention is a genetically modified organism of the present invention, i.e. having a chemolithotrophic and/or methylotrophic metabolism, and/or is a genetically modified organism having a heterotrophic metabolism. In one embodiment, a genetically modified organism of the present invention is capable of a chemolithotrophic and/or methylotrophic metabolism, and is also capable of a heterotrophic metabolism.

The term "culturing", as used herein, relates to growing cells, preferably under controlled conditions. Genetically modified organisms of the present invention are preferably cultured at a temperature of from 25 to 60 °C for a period of 12 to 96 hours. In one embodiment, said genetically modified organism is *C. necator* and is cultured at a temperature of from 25 °C to 35 °C for a period of 12 to 96 hours.

The term "supplying", as used herein with regard to substrates, relates to providing substrates for growth and biosynthesis to cells in a cell culture. In one embodiment, a method of producing trehalose and/or glucose involves a substrate, such as a carbon source, which is solid or gaseous. In one embodiment, a gaseous substrate is obtained from waste biomass and/or biogas. In one embodiment, using a gaseous substrate such as a gaseous mixture comprising H₂, O₂, and CO₂ allows for an efficient production of sugars. In one embodiment, using a synthesis gas comprising CO₂ is environmentally friendly since CO₂ is fixed. In one embodiment, using a synthesis gas comprising H₂ is advantageous in that H₂ is available from numerous resources such as electrolysis, natural gas, and gasification of waste biomass. In one embodiment, a method of the present invention is advantageous in that it is allows for a cost-efficient production of an expensive product, which is isotope-labelled trehalose and/or isotope-labelled glucose, from readily available isotope-labelled ¹³CO₂. In one embodiment, a method of producing isotope-labelled trehalose and/or isotope-labelled glucose comprises, in step ii), supplying a mixture of ¹³CO₂, nitrate, H₂, and/or supplying a mixture of ¹³CO₂, phosphite, O₂, and/or supplying a mixture of ¹³CO₂, O₂, H₂, preferably comprises supplying a mixture of ¹³CO₂, O₂, and H₂. In one embodiment, in a method of the present invention, a genetically modified organism is preferably supplied with a substrate which comprises a gaseous mixture comprising H₂, O₂, and CO₂. In one embodiment, the term "comprising" may relate to "consisting of'.

The term "inorganic electron source", as used herein, relates to an inorganic substrate as a source of electron donors to drive energy acquisition, for example a substrate for growth and biosynthesis of an organism having a chemolithotrophic and/or methylotrophic metabolism. In one embodiment, an inorganic electron source is any of phosphite, divalent iron, ammonia, nitrite, sulfide, sulfur, hydrogen, ammonium, carbon monoxide, mixtures thereof, and synthesis gas, such as a gaseous mixture comprising H₂, O₂, and CO₂, preferably any of phosphite, nitrite, hydrogen, ammonium, sulfide, synthesis gas, and mixtures thereof. In one embodiment, synthesis gas comprising H₂, O₂, and CO₂ is provided in a mixture having a ratio (v/v) of 6:1:1, respectively, and/or in a mixture comprising 20-99% (v/v) H₂, 0.1-20% (v/v) O₂, and 0.1-20% (v/v) CO₂. In one embodiment, synthesis gas is obtained from gasification of biomass, such as waste material. In one embodiment, an inorganic electron source is derived from biomass which is burned to provide a gaseous substrate mixture comprising any of H₂, CO₂, CO, H₂S, and NH₃. In one embodiment, synthesis gas comprises H₂, CO₂, CO, H₂S, and NH₃, and/or H₂, CO₂, and O₂. In one embodiment, organisms having a chemolithotrophic and/or methylotrophic metabolism are capable of using synthesis gas as a substrate. In one embodiment, if phosphite is supplied as a substrate, phosphite is supplied in the form of a salt, such as Na₂HPO₃·5H₂O, or in the form of a liquid.

The term "obtaining trehalose and/or glucose from said cell culture", as used herein, relates to obtaining trehalose and/or glucose from the supernatant of the cell culture and/or from lysed cells. In one embodiment, said trehalose and/or glucose is/are secreted from said genetically modified microorganism and is/are obtained from a supernatant of a cell culture of said genetically modified microorganism. In one embodiment, obtaining trehalose and/or glucose may comprise any of centrifugation, filtration, chromatography, and crystallization. In one embodiment, obtaining of glucose comprises enzymatic cleavage of trehalose by trehalase to obtain glucose. In one embodiment, an organism such as *E. coli* or *C. necator* has an intracellular trehalase, optionally a periplasmatic trehalase, that cleaves trehalase to glucose. In one embodiment, a genetically modified organism is genetically modified to (over)express a trehalase. In one embodiment, glucose is produced by secretion of glucose via a sugar efflux transporter such as SetA and subsequent cleavage of trehalose by a trehalase, such as a periplasmatic trehalase, a secreted trehalase, and/or a trehalase added to the cell culture medium. In an alternative embodiment, glucose is produced by intracellular cleavage of trehalose by an intracellular trehalase, e.g. TreF of *E. coli,* and secretion of glucose produced thereby via a sugar efflux transporter such as SetA or Glf. The term "Glf' relates to glucose facilitated diffusion protein of *Zymomonas mobilis.*

The term "isotope-labelled trehalose and/or isotope-labelled glucose", as used herein, relates to trehalose and/or glucose, respectively, that are labelled with an isotope, such as C¹³. A method of the present invention is advantageous in that it allows to easily and efficiently produce isotope-labelled trehalose and/or isotope-labelled glucose which are valuable for isotopic markers, e.g. in medicine. Thereby, isotope-labelled sugar can be produced in a cost- and resource-efficient manner.

In one embodiment, if said method is a method of producing isotope-labelled trehalose and/or isotope-labelled glucose, said supplying preferably comprises H₂, O₂, and isotope-labelled CO₂.

In one embodiment, in a method of producing of the present invention, the genetically modified organism is subjected to salt stress for increasing the production and/or secretion of trehalose and/or glucose, i.e. culturing the genetically modified organism comprises subjecting said organism to a salt concentration of at least 50 mM salt, preferably a salt concentration in a range of from 20 mM to 190 mM, more preferably about 150 mM. In one embodiment, the salt is preferably NaCl. In one embodiment, a method of the present invention is advantageous in that optionally two effects are used to efficiently provide trehalose and/or glucose, namely overexpressing a sugar transporter, such as SetA as a trehalose transport protein, and optionally increasing trehalose accumulation in a cell under salt stress.

In one embodiment, a method of the present invention achieves a trehalose concentration of at least 0,5 g/L, preferably at least 10 g/L, more preferably of at least 50 g/L. in one embodiment, said trehalose is obtained from the cell culture medium and/or the cells. In one embodiment, a concentration of trehalose and/or glucose is measured using any of HPLC, an enzymatic assay, and a photometric analysis.

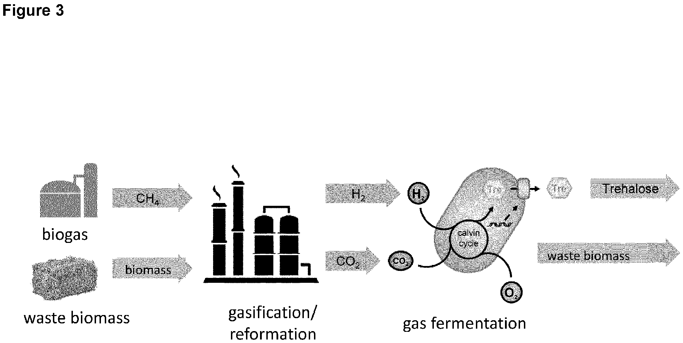

In one embodiment, the waste microorganism biomass (Figure 3) obtained after fermentation in a method of the present invention can be used as animal food or as fertilizer.

The term "sugar", as used herein, relates to any carbohydrate such as monosaccharides, disaccharides, oligosaccharides, and polysaccharides. Exemplary monosaccharides include glucose, fructose, and galactose, and exemplary disaccharides include sucrose (glucose + fructose), lactose (glucose + galactose), trehalose (two molecules of glucose), and maltose (two molecules of glucose). In one embodiment, a sugar is any of trehalose, glucose, glycogen, saccharose, isomaltose, isomaltulose, glucosylglycerol, kojibiose, and cellobiose, preferably is any of trehalose and glucose. In many of the embodiments, when referring to the terms "trehalose" and "glucose", the terms also comprise "isotope-labelled trehalose" and "isotope-labelled glucose", respectively.

### BRIEF DESCRIPTION OF THE FIGURES

The present invention is now further described by reference to the following figures. All methods mentioned in the figure descriptions below were carried out as described in detail in the examples.
**Figure 1** shows a schematic representation of a method of the present invention on a cellular level. SetA is an exemplary sugar efflux transporter A from *E. coli.* An exemplary genetically modified organism of the present invention is *Cupriavidus necator* H16. An exemplary organism metabolizes synthesis gas comprising H₂/CO₂/O₂ via the Calvin cycle.
**Figure 2** shows a growth experiment of an exemplary genetically modified organism which is a *C. necator* H16 strain.
   A) OD as a measure of cell growth over time.
   B) Trehalose concentration over time.
   The experiments were conducted in a MR minimal medium with H₂/CO₂/O₂ as the sole carbon and energy source. Cultures were grown in butyl-rubber sealed bottles with 10% liquid and 90% gas phase at an overpressure of 0.14 bar. The exemplary organism is able to grow using synthesis gas comprising H₂/CO₂/O₂ as the sole carbon and energy source. Furthermore, trehalose is efficiently produced from a mixture of H₂/CO₂/O₂ as the substrate. The production of trehalose is further enhanced by addition of NaCl to the medium.
**Figure 3** shows a schematic representation of one embodiment of a method of the present invention. Residual biomass from agriculture, such as straw, is thermochemically converted into synthesis gas. Alternatively, biogas can be converted into H₂ and O₂ by steam reforming and/or H₂, CO, and CO₂. Subsequently, the respective gas is fermented into trehalose by a genetically modified organism according to the present invention. The biomass obtained from said microorganisms can be used as animal food or as a fertilizer.

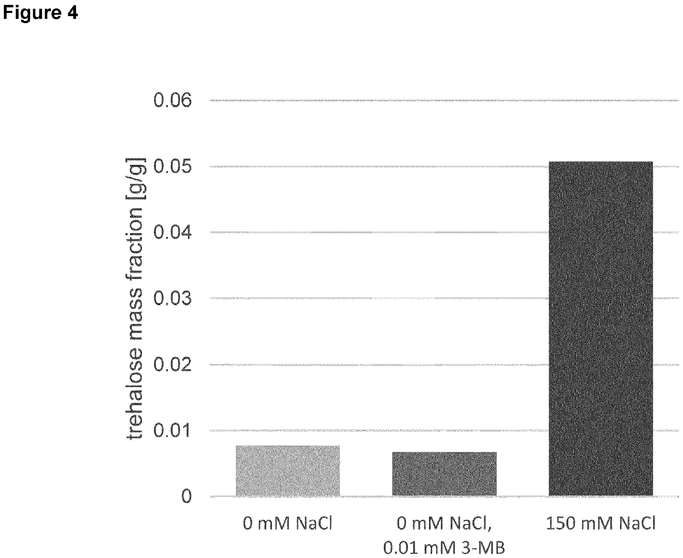

**Figure 4** shows mass fractions of intracellular trehalose of *C. necator* cultures grown in minimal medium with fructose at different salt concentrations. Trehalose is efficiently produced by the organism. The effect of the substance 3-methylbezoate (3-MB) on the accumulation of trehalose was tested as a control to exclude an influence of the inducer when using XylS/pm based expression plasmids. Mass fractions of up to 5% of the dry weight were achieved at a salt concentration of 150 mM NaCl.

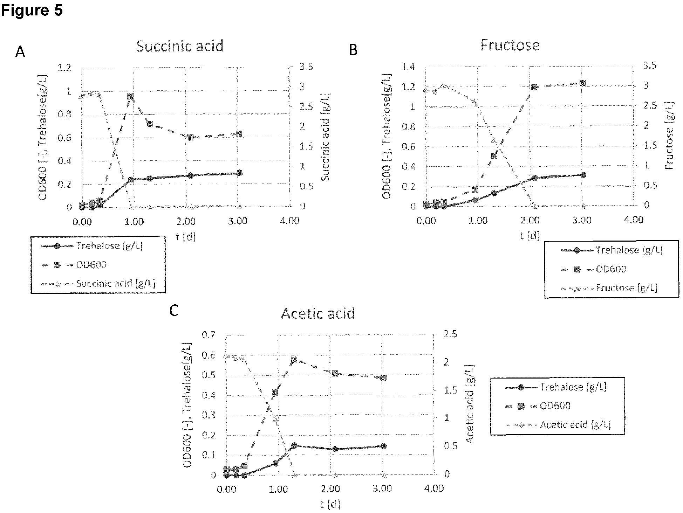

**Figure 5** shows efficient trehalose production from carbon sources fructose, acetic acid, and succinic acid. A genetically modified organism of the present invention and a method of the present invention allow to produce and secrete sugars, such as trehalose, from organic carbon sources such as succinic acid (A), fructose (B), and acetic acid (C).

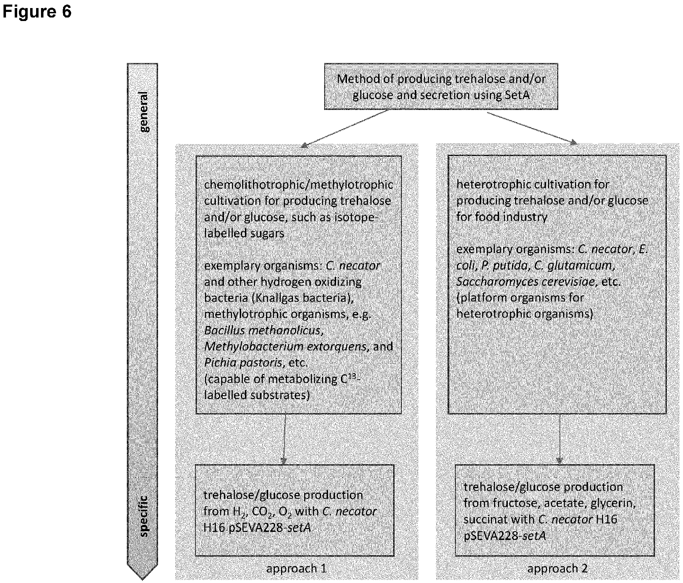

**Figure 6** shows a flow chart of exemplary methods of the present invention for trehalose/glucose production from inorganic substrates and/or organic carbon substrates. A method of the present invention involves a chemolithotrophic, methylotrophic and/or heterotrophic cultivation of genetically modified organisms expressing a sugar efflux transporter. In one embodiment, a method of the present invention comprises chemolithotrophic cultivation with a substrate such as H₂ and/or phosphite, and/or comprises methylotrophic cultivation with a substrate such as CO, methanol, and/or formic acid, and/or comprises heterotrophic cultivation with a substrate such as glycerol and/or acetic acid. In one embodiment, *C. necator* H16 is capable of a chemolithotrophic metabolism, such as using any substrate of H₂, CO₂, and O₂, and is capable of a methylotrophic metabolism, such as using formic acid, and is capable of a heterotrophic metabolism, such as using fructose.

In the following, reference is made to the examples, which are given to illustrate, not to limit the present invention.

### EXAMPLES

### Example 1: Cultivation of C. necator H16 pSEVA228-setA

A 3-5 mL starter culture of lysogeny broth (LB) medium with 2.5 g/L NaCl (instead of 10 g/L in the original recipe), supplemented with 300 mg/L kanamycin in a cultivation vial was prepared in bottles or shaking flasks. A colony of *C. necator H16* pSEVA228-*setA* from a fresh plate was taken for inoculation and the vial was incubated for 18 h at 30°C and 220 rpm in a shaking incubator.

For the main culture, MR-medium supplemented with a trace-element solution was used. MR medium contains per liter of medium: (NH₄)₂HPO₄, 4 g; KH₂PO₄, 6.67 g; MgSO₄·3H₂O, 0.8 g. The present inventors added 300 mg/L kanamycin, 0-200 mM of NaCl, 0.1 mM of 3-methylbenzoate, 1 mL/L of the BG-11 trace element solution and either a suitable carbon source or a gas mixture containing H₂/CO₂/O₂-mixture of 6:1:1. For gas cultivation experiments, rubber sealed bottles with a liquid to gas ratio of 1:20 were used.

The main cultures were inoculated with a volume equal to 1-4% of the main culture volume with the LB starter culture and then inoculated at 30°C and 220 rpm in a shaking incubator. OD was followed with a photometer and trehalose concentration was determined by HPLC with a Shodex SH1011 column, after trehalase treatment of the samples.
For the trehalase treatment, a raw lysate of *Escherichia coli* DH5α expressing its native trehalase TreF from a pSEVA228 plasmid (SEQ ID NO. 6) was used. For production of TreF, *E. coli* was grown in LB medium with 50 µg/mL kanamycin and 0.1 mM 3-methylbenzoate. After reaching stationary phase, cells were centrifuged, up-concentrated to an OD₆₀₀ of approximately 50, resuspended in phosphate-buffered saline (PBS) and mechanically disrupted in a ball mill for 20 min. The raw lysate was added to the trehalose containing sample, constituting 10% of the whole volume. The mixture was incubated for 4 hours at 37°C, inactivated and prepared for HPLC measurement for measuring the resulting glucose concentrations.

### Example 2: Trehalose production from a gaseous substrate

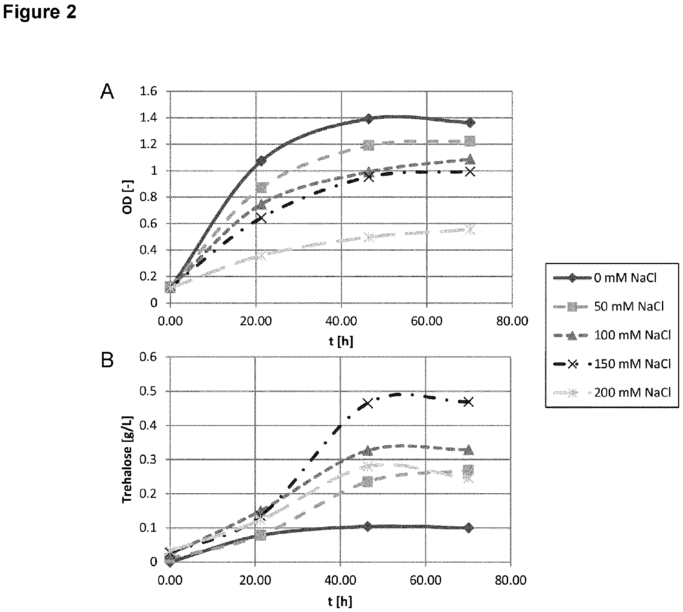

*C. necator* H16 pSEVA228-*setA* was cultured as described in Example 1 in a main culture with a H₂/CO₂/ O₂ mixture of 6:1:1 with 150 mM NaCl in butyl-rubber sealed bottles. Expression of the plasmid pSEVA228-setA was induced with 0.1 mM 3-methyl benzoate and samples were taken with a syringe through the rubber sealing. Trehalose content in the medium and OD were followed by HPLC and photometry, respectively. The cells start to produce up to ∼0.5 g/L trehalose from the gases (Figure 2) over the course of three days. For this final titer, the natural production capability of trehalose by *C. necator* was sufficient. Over-production of trehalose synthesis genes such as phospho-glucoisomerase, UTP-glucose-1-phosphate uridylyltransferase, trehalose phosphate synthetase and trehalose-phosphate phosphatase enhances the trehalose yield and production rate. Moreover, trehalose production seemed to be growth-coupled. By supplying more substrate and/or artificially limiting cell growth, the present inventors expect higher magnitudes of trehalose.

### Example 3: Trehalose production from fructose

*C. necator* cultures were grown in minimal medium with 3 g/L fructose at different salt concentrations. The cells were lysed in the stationary phase by mechanical disruption in a ball mill and the cell content of trehalose was analyzed using HPLC. High amounts of trehalose were intracellularly accumulated. Mass fractions of up to 5% of the dry weight were achieved at a salt concentration of 150 mM NaCl (Figure 4).

### Example 4: Trehalose production from succinic acid, acetic acid, and fructose

*C. necator* H16 pSEVA228-*setA* can also be used to produce trehalose from organic substrates. In this case, cells are cultured as described in Example 1 and fructose, acetate or succinate were used as organic carbon sources for the main culture with a concentration of 3 g/L, 2.1 g/L and 3 g/L. Trehalose and substrate content in the medium and OD were measured using HPLC and photometry, respectively (Fig. 5). Trehalose was produced and secreted during the whole growth phase and could be easily collected from the culture supernatant. Glycerol, as a cheap carbon source, was also tested and led to a comparable trehalose secretion (data not shown) albeit after a lag time of more than one week.

### REFERENCES

[1] Cai X, Seitl I, Mu W et al. Appl Microbiol Biotechnol (2018) 102: 2965. https://doi.org/10.1007/s00253-018-8814-y.
[2] Löwe H, Schmauder L, Hobmeier K, Kremling A, Pflüger-Grau K et al. Microbiology Open (2017) 6: 4. e00473. https://doi.org/10.1002/mbo3.473.
[3] Kizawa H, Miyazaki J, Yokota A, Kanegae Y, Miyagawa K, Sugiyama Y. Bioscience, biotechnology, and biochemistry (1995) 59:8, 1522-1527. DOI: 10.1271/bbb.59.1522.
[4] Gonzalez-Villanueva, M., Galaiya, H., Staniland, P. et al. (2019) Adaptive laboratory evolution of cupriavidus necator H16 for carbon co-utilization with glycerol. International Journal of Molecular Sciences, 20 (22). 5737. ISSN 1661-6596.
[5] Nangle S, Ziesack M, Buckley S, Trivedi D, Loh D, et al. (2020) Valorization of CO2 through lithoautotrophic production of sustainable chemicals in Cupriavidus necator. BioRxiv 2020.02.08.940007. https://doi.org/10.1101/2020.02.08.940007.

The features of the present invention disclosed in the specification, the claims, and/or in the accompanying figures may, both separately and in any combination thereof, be material for realizing the invention in various forms thereof.

## Claims

1. A genetically modified organism, wherein said organism is selected from organisms having a chemolithotrophic and/or methylotrophic metabolism, wherein said organism expresses a sugar efflux transporter, preferably a sugar uniporter or a cation/sugar antiporter, optionally further overexpresses one or more trehalose synthesis gene(s).

2. The genetically modified organism according to claim 1, wherein said expression, optionally overexpression, comprises an expression of an exogenous gene of said sugar efflux transporter, optionally of said one or more trehalose synthesis gene(s), in said organism, and/or comprises an increased expression of an endogenous gene of said sugar efflux transporter, optionally of said one or more trehalose synthesis gene(s), in said organism.

3. The genetically modified organism according to claims 1 or 2, wherein said organism is a chemolithotrophic organism, preferably selected from hydrogen oxidizing bacteria, autotrophic phosphite oxidizing bacteria, autotrophic nitrate/nitrite or sulfate respiring microorganisms, and genetically modified organisms having obtained chemolithotrophic characteristics by genetic modification, more preferably *Cupriavidus necator.*

4. The genetically modified organism according to any of the foregoing claims, wherein said organism is a methylotrophic organism, preferably any organism selected from Cupriavidus sp., such as *Cupriavidus necator H16,* Bacillus sp., such as *Bacillus methanolicus,* Methylobacterium sp., such as *Methylobacterium extorquens,* Hydrogenophaga sp., such as *Hydrogenophaga pseudoflava,* Rhodococcus sp., such as *Rhodococcus opacus,* Ralstonia sp., Pichia sp., such as *Pichia pastoris,* Komagatella sp., and Paracoccus sp., such as *Paracoccus denitrificans,* and genetically modified organisms having obtained methylotrophic characteristics by genetic modification.

5. The genetically modified organism according to any of the foregoing claims, wherein said sugar efflux transporter is any of a sugar uniporter, a H⁺ or Na⁺-antiporter, and/or an ATP-dependent efflux transport protein, preferably is a sugar uniporter or H⁺/sugar-antiporter selected from the sugar efflux transporter A (SetA) from *Escherichia coli* (SEQ ID NO. 1), the glucose facilitator Glf from *Zymomonas mobilis* (SEQ ID NO. 2), the transport protein YdeA from *E. coli* (SEQ ID NO. 3), a protein of the SWEET transport protein family, the Tret1-1 trehalose facilitator from *Drosophila melanogaster* (SEQ ID NO. 4), a protein from the (GLUT)/SLC2A transport protein family, and homologues and variants thereof, more preferably is SetA.

6. The genetically modified organism according to claim 5, wherein SetA is derived from *E. coli,* preferably SetA having SEQ ID NO. 1, and/or is a SetA homologue or variant.

7. The genetically modified organism according to any of the foregoing claims, wherein said one or more trehalose synthesis gene(s) are selected from phosphoglucosemutase, UTP-glucose-i-phosphate uridylyltransferase, trehalose phosphate synthetase, trehalose-phosphate phosphatase, maltooligosyl trehalose synthase, and maltooligosyl trehalose hydrolase.

8. The genetically modified organism according to any of the foregoing claims, wherein said organism is transformed with pSEVA228-*setA* having SEQ ID NO. 5.

9. The genetically modified organism according to any of the foregoing claims, wherein said organism is *Cupriavidus necator* Cn_H16_*setA* deposited at the Deutsche Sammlung von Mikroorganismen und Zellkulturen GmbH having accession number DSM 33415.

10. A method of producing trehalose and/or glucose, wherein said method comprises the following steps:
i) providing a genetically modified organism as defined in any of claims 1-9 and/or a genetically modified organism expressing a sugar efflux transporter, preferably SetA, optionally further overexpressing one or more trehalose synthesis gene(s);
ii) culturing the genetically modified organism of step i) in a cell culture, said culturing comprising supplying an inorganic electron source, preferably H₂ in a gaseous mixture comprising H₂, O₂, and CO₂, and/or phosphite, and/or supplying any of nitrate as electron acceptor, carbonate, or an organic carbon source, such as fructose, succinic acid, acetic acid, glycerol, and mixtures thereof; and
iii) obtaining trehalose and/or glucose from said cell culture.

11. The method according to claim 10, wherein, in step iii), said trehalose and/or glucose is/are obtained from a supernatant of said cell culture.

12. The method according to claims 10 or 11, wherein, in step ii), said gaseous mixture comprises 20-99% (v/v) H₂, 0.1-20% (v/v) O₂, and 0.1-20% (v/v) CO₂.

13. The method according to any of claims 10-12, wherein, in step ii), said H₂, O₂, and CO₂ are supplied in a ratio (v/v) of 6:1:1, respectively, to said cell culture.

14. The method according to any of claims 10-13, wherein, in step iii), said obtaining of glucose further comprises enzymatic cleavage of trehalose by trehalase to obtain glucose.

15. The method according to any of claims 10-14, wherein said method is a method of producing isotope-labelled trehalose and/or isotope-labelled glucose, said method comprising
in step ii), supplying an inorganic electron source, preferably H₂ in a gaseous mixture comprising H₂, isotope-labelled CO₂, and O₂, and/or phosphite, and/or supplying nitrate as an electron acceptor, and/or supplying any of isotope-labelled carbonate, fructose, succinic acid, acetic acid, glycerol, and mixtures thereof, and,
in step iii), obtaining isotope-labelled trehalose and/or isotope-labelled glucose, preferably isotope-labelled glucose.

16. Use of a genetically modified organism, as defined in any of claims 1-9, for producing trehalose and/or glucose, optionally isotope-labelled trehalose and/or isotope labelled glucose.


SEQUENCE LISTING

<110> Technische Universität München

<120> A new method for the production of trehalose from gaseous substrates

<130> U30960EP

<160> 6

<170> BiSSAP 1.3.6

<210> 1
<211> 392
<212> PRT
<213> Escherichia coli

<220>
<223> SetA

<400> 1
Met Ile Trp Ile Met Thr Met Ala Arg Arg Met Asn Gly Val Tyr Ala
1               5                   10                  15
Ala Phe Met Leu Val Ala Phe Met Met Gly Val Ala Gly Ala Leu Gln
            20                  25                  30
Ala Pro Thr Leu Ser Leu Phe Leu Ser Arg Glu Val Gly Ala Gln Pro
        35                  40                  45
Phe Trp Ile Gly Leu Phe Tyr Thr Val Asn Ala Ile Ala Gly Ile Gly
    50                  55                  60
Val Ser Leu Trp Leu Ala Lys Arg Ser Asp Ser Gln Gly Asp Arg Arg
65                  70                  75                  80
Lys Leu Ile Ile Phe Cys Cys Leu Met Ala Ile Gly Asn Ala Leu Leu
                85                  90                  95
Phe Ala Phe Asn Arg His Tyr Leu Thr Leu Ile Thr Cys Gly Val Leu
            100                 105                 110
Leu Ala Ser Leu Ala Asn Thr Ala Met Pro Gln Leu Phe Ala Leu Ala
        115                 120                 125
Arg Glu Tyr Ala Asp Asn Ser Ala Arg Glu Val Val Met Phe Ser Ser
    130                 135                 140
Val Met Arg Ala Gln Leu Ser Leu Ala Trp Val Ile Gly Pro Pro Leu
145                 150                 155                 160
Ala Phe Met Leu Ala Leu Asn Tyr Gly Phe Thr Val Met Phe Ser Ile
                165                 170                 175
Ala Ala Gly Ile Phe Thr Leu Ser Leu Val Leu Ile Ala Phe Met Leu
            180                 185                 190
Pro Ser Val Ala Arg Val Glu Leu Pro Ser Glu Asn Ala Leu Ser Met
        195                 200                 205
Gln Gly Gly Trp Gln Asp Ser Asn Val Arg Met Leu Phe Val Ala Ser
    210                 215                 220
Thr Leu Met Trp Thr Cys Asn Thr Met Tyr Ile Ile Asp Met Pro Leu
225                 230                 235                 240
Trp Ile Ser Ser Glu Leu Gly Leu Pro Asp Lys Leu Ala Gly Phe Leu
                245                 250                 255
Met Gly Thr Ala Ala Gly Leu Glu Ile Pro Ala Met Ile Leu Ala Gly
            260                 265                 270
Tyr Tyr Val Lys Arg Tyr Gly Lys Arg Arg Met Met Val Ile Ala Val
        275                 280                 285
Ala Ala Gly Val Leu Phe Tyr Thr Gly Leu Ile Leu Phe His Ser Arg
    290                 295                 300
Leu Ala Leu Met Thr Leu Gln Leu Phe Asn Ala Val Phe Ile Gly Ile
305                 310                 315                 320
Val Ala Gly Ile Gly Met Leu Trp Phe Gln Asp Leu Met Pro Gly Arg

325                 330                 335
Ala Gly Ala Ala Thr Thr Leu Phe Thr Asn Ser Ile Ser Thr Gly Val
            340                 345                 350
Ile Leu Ala Gly Val Ile Gln Gly Ala Ile Ala Gln Ser Trp Gly His
        355                 360                 365
Phe Ala Val Tyr Trp Val Ile Ala Val Ile Ser Val Ile Ala Leu Phe
    370                 375                 380
Leu Thr Ala Lys Val Lys Asp Val
385                 390

<210> 2
<211> 473
<212> PRT
<213> Zymomonas mobilis

<220>
<223> Glf

<400> 2
Met Ser Ser Glu Ser Ser Gln Gly Leu Val Thr Arg Leu Ala Leu Ile
1               5                   10                  15
Ala Ala Ile Gly Gly Leu Leu Phe Gly Tyr Asp Ser Ala Val Ile Ala
            20                  25                  30
Ala Ile Gly Thr Pro Val Asp Ile His Phe Ile Ala Pro Arg His Leu
        35                  40                  45
Ser Ala Thr Ala Ala Ala Ser Leu Ser Gly Met Val Val Val Ala Val
    50                  55                  60
Leu Val Gly Cys Val Thr Gly Ser Leu Leu Ser Gly Trp Ile Gly Ile
65                  70                  75                  80
Arg Phe Gly Arg Arg Gly Gly Leu Leu Met Ser Ser Ile Cys Phe Val
                85                  90                  95
Ala Ala Gly Phe Gly Ala Ala Leu Thr Glu Lys Leu Phe Gly Thr Gly
            100                 105                 110
Gly Ser Ala Leu Gln Ile Phe Cys Phe Phe Arg Phe Leu Ala Gly Leu
        115                 120                 125
Gly Ile Gly Val Val Ser Thr Leu Thr Pro Thr Tyr Ile Ala Glu Ile
    130                 135                 140
Ala Pro Pro Asp Lys Arg Gly Gln Met Val Ser Gly Gln Gln Met Ala
145                 150                 155                 160
Ile Val Thr Gly Ala Leu Thr Gly Tyr Ile Phe Thr Trp Leu Leu Ala
                165                 170                 175
His Phe Gly Ser Ile Asp Trp Val Asn Ala Ser Gly Trp Cys Trp Ser
            180                 185                 190
Pro Ala Ser Glu Gly Leu Ile Gly Ile Ala Phe Leu Leu Leu Leu Leu
        195                 200                 205
Thr Ala Pro Asp Thr Pro His Trp Leu Val Met Lys Gly Arg His Ser
    210                 215                 220
Glu Ala Ser Lys Ile Leu Ala Arg Leu Glu Pro Gln Ala Asp Pro Asn
225                 230                 235                 240
Leu Thr Ile Gln Lys Ile Lys Ala Gly Phe Asp Lys Ala Met Asp Lys
                245                 250                 255
Ser Ser Ala Gly Leu Phe Ala Phe Gly Ile Thr Val Val Phe Ala Gly
            260                 265                 270
Val Ser Val Ala Ala Phe Gln Gln Leu Val Gly Ile Asn Ala Val Leu
        275                 280                 285
Tyr Tyr Ala Pro Gln Met Phe Gln Asn Leu Gly Phe Gly Ala Asp Thr
    290                 295                 300
Ala Leu Leu Gln Thr Ile Ser Ile Gly Val Val Asn Phe Ile Phe Thr
305                 310                 315                 320
Met Ile Ala Ser Arg Val Val Asp Arg Phe Gly Arg Lys Pro Leu Leu
                325                 330                 335
Ile Trp Gly Ala Leu Gly Met Ala Ala Met Met Ala Val Leu Gly Cys

340                 345                 350
Cys Phe Trp Phe Lys Val Gly Gly Val Leu Pro Leu Ala Ser Val Leu
        355                 360                 365
Leu Tyr Ile Ala Val Phe Gly Met Ser Trp Gly Pro Val Cys Trp Val
    370                 375                 380
Val Leu Ser Glu Met Phe Pro Ser Ser Ile Lys Gly Ala Ala Met Pro
385                 390                 395                 400
Ile Ala Val Thr Gly Gln Trp Leu Ala Asn Ile Leu Val Asn Phe Leu
                405                 410                 415
Phe Lys Val Ala Asp Gly Ser Pro Ala Leu Asn Gln Thr Phe Asn His
            420                 425                 430
Gly Phe Ser Tyr Leu Val Phe Ala Ala Leu Ser Ile Leu Gly Gly Leu
        435                 440                 445
Ile Val Ala Arg Phe Val Pro Glu Thr Lys Gly Arg Ser Leu Asp Glu
    450                 455                 460
Ile Glu Glu Met Trp Arg Ser Gln Lys
465                 470

<210> 3
<211> 395
<212> PRT
<213> Escherichia coli

<220>
<223> YdeA

<400> 3
Met Asn Leu Ser Leu Arg Arg Ser Thr Ser Ala Leu Leu Ala Ser Ser
1               5                   10                  15
Leu Leu Leu Thr Ile Gly Arg Gly Ala Thr Leu Pro Phe Met Thr Ile
            20                  25                  30
Tyr Leu Ser Arg Gln Tyr Ser Leu Ser Val Asp Leu Ile Gly Tyr Ala
        35                  40                  45
Met Thr Ile Ala Leu Thr Ile Gly Val Val Phe Ser Leu Gly Phe Gly
    50                  55                  60
Ile Leu Ala Asp Lys Phe Asp Lys Lys Arg Tyr Met Leu Leu Ala Ile
65                  70                  75                  80
Thr Ala Phe Ala Ser Gly Phe Ile Ala Ile Thr Leu Val Asn Asn Val
                85                  90                  95
Thr Leu Val Val Leu Phe Phe Ala Leu Ile Asn Cys Ala Tyr Ser Val
            100                 105                 110
Phe Ala Thr Val Leu Lys Ala Trp Phe Ala Asp Asn Leu Ser Ser Thr
        115                 120                 125
Ser Lys Thr Lys Ile Phe Ser Ile Asn Tyr Thr Met Leu Asn Ile Gly
    130                 135                 140
Trp Thr Ile Gly Pro Pro Leu Gly Thr Leu Leu Val Met Gln Ser Ile
145                 150                 155                 160
Asn Leu Pro Phe Trp Leu Ala Ala Ile Cys Ser Ala Phe Pro Met Leu
                165                 170                 175
Phe Ile Gln Ile Trp Val Lys Arg Ser Glu Lys Ile Ile Ala Thr Glu
            180                 185                 190
Thr Gly Ser Val Trp Ser Pro Lys Val Leu Leu Gln Asp Lys Ala Leu
        195                 200                 205
Leu Trp Phe Thr Cys Ser Gly Phe Leu Ala Ser Phe Val Ser Gly Ala
    210                 215                 220
Phe Ala Ser Cys Ile Ser Gln Tyr Val Met Val Ile Ala Asp Gly Asp
225                 230                 235                 240
Phe Ala Glu Lys Val Val Ala Val Val Leu Pro Val Asn Ala Ala Met
                245                 250                 255
Val Val Thr Leu Gln Tyr Ser Val Gly Arg Arg Leu Asn Pro Ala Asn
            260                 265                 270
Ile Arg Ala Leu Met Thr Ala Gly Thr Leu Cys Phe Val Ile Gly Leu

275                 280                 285
Val Gly Phe Ile Phe Ser Gly Asn Ser Leu Leu Leu Trp Gly Met Ser
    290                 295                 300
Ala Ala Val Phe Thr Val Gly Glu Ile Ile Tyr Ala Pro Gly Glu Tyr
305                 310                 315                 320
Met Leu Ile Asp His Ile Ala Pro Pro Glu Met Lys Ala Ser Tyr Phe
                325                 330                 335
Ser Ala Gln Ser Leu Gly Trp Leu Gly Ala Ala Ile Asn Pro Leu Val
            340                 345                 350
Ser Gly Val Val Leu Thr Ser Leu Pro Pro Ser Ser Leu Phe Val Ile
        355                 360                 365
Leu Ala Leu Val Ile Ile Ala Ala Trp Val Leu Met Leu Lys Gly Ile
    370                 375                 380
Arg Ala Arg Pro Trp Gly Gln Pro Ala Leu Cys
385                 390                 395

<210> 4
<211> 857
<212> PRT
<213> Drosophila melanogaster


<220>
<223> Tret1-1

<400> 4
Met Ser Gly Arg Asp Asn Arg Gly Ala Gly Gly Gly Gly Gly Gly His
1               5                   10                  15
Gln Pro Leu Ser Asn Ala Met Gly Lys Leu Lys Glu Lys Leu Thr Arg
            20                  25                  30
Val Gly Asp Glu Leu Gly Tyr His Arg Val Glu Ser Asn Leu Ser Thr
        35                  40                  45
Ser Asn Thr Ala Thr Ser Leu Asp Thr Ile Leu Pro Glu Asp Pro Phe
    50                  55                  60
Leu Phe Pro Gln Val Ser Pro Gln Arg His Pro Gln Asn Thr Val Arg
65                  70                  75                  80
Thr Gln Arg Leu Leu Glu Asp Glu Pro Pro Leu Ser Phe Arg Pro Leu
                85                  90                  95
Leu Glu Asp Asp Asp Ile Asn Glu Pro Pro Thr Gln Gln Gln Gln Arg
            100                 105                 110
Thr Pro Leu Arg Ala Ser Gly Ser Leu Glu Leu Thr Pro Leu Pro Pro
        115                 120                 125
Pro Pro Thr Ser Leu Glu Ile Arg Glu His Arg Asp Arg Gln Gln Arg
    130                 135                 140
Gly Ala Gln Gly Asp Glu Leu Gln Arg Ser Lys Gln Ser Leu Lys Gly
145                 150                 155                 160
Ser Arg Val Ser Phe Glu Arg Arg Asp Thr Gly Asn Ser Asn Thr Asn
                165                 170                 175
Ser Asn Lys Ala Ala Glu Ser Ser Asp Glu Asp Ser Phe Glu Glu Lys
            180                 185                 190
Arg Thr Gly Phe Gln Gln Gln Lys Ala Thr Ser Val Asp His Lys Gly
        195                 200                 205
Ile Leu Lys Asp Leu Lys His Ile Leu Ala Asn Asp Asn Arg Arg Gln
    210                 215                 220
Phe Gln Ala Lys Lys His Val Ser Leu Asp Val Lys Gly Thr Arg Phe
225                 230                 235                 240
Leu Gln Asp Leu Leu Lys Glu Ser Ser Ser Glu Glu Glu Phe His Lys
                245                 250                 255
Thr Arg Arg Glu Phe Gln Gly Arg Lys His Gln Ser Leu Asp Pro Arg
            260                 265                 270
Val Thr Phe Lys Leu Asp Lys Val Leu Gln Gly Ser Ser Thr Asp Ser
        275                 280                 285
Asp Glu Glu Gly Glu Asp Ala Glu His Lys Arg Leu Ile His Arg Pro

290                 295                 300
Lys Asp Ile Thr Lys Pro Val Ile Ile Asp Leu Lys Asp Leu Glu Ser
305                 310                 315                 320
Glu Ser Asp Glu Asp Phe Leu Thr Ser Arg Gln His Phe Gln Gln Gln
                325                 330                 335
Arg Ser Ile Ser Thr Asp Ser Arg Lys Ser Arg Arg Leu Tyr Glu Met
            340                 345                 350
Asp Glu Met Asp Asn Lys Arg Gly Glu Asn Ile Arg His Ala Val Pro
        355                 360                 365
Phe Val Arg Gln Ile Thr Glu Asp Gly Lys Pro Lys Leu Glu Val Tyr
    370                 375                 380
Arg Pro Thr Thr Asn Pro Ile Tyr Ile Trp Thr Gln Val Leu Ala Ala
385                 390                 395                 400
Leu Ser Val Ser Leu Gly Ser Leu Val Val Gly Phe Val Ser Ala Tyr
                405                 410                 415
Thr Ser Pro Ala Leu Val Ser Met Thr Asp Arg Asn Ile Thr Ser Phe
            420                 425                 430
Glu Val Thr Gln Asp Ala Gly Ser Trp Val Gly Gly Ile Met Pro Leu
        435                 440                 445
Ala Gly Leu Ala Gly Gly Ile Ala Gly Gly Pro Leu Ile Glu Tyr Leu
    450                 455                 460
Gly Arg Arg Asn Thr Ile Leu Ala Thr Ala Val Pro Phe Ile Val Ser
465                 470                 475                 480
Ser Leu Leu Ile Ala Cys Ala Val Asn Val Ala Met Val Leu Cys Gly
                485                 490                 495
Arg Phe Leu Ala Gly Phe Cys Val Gly Ile Ala Ser Leu Ser Leu Pro
            500                 505                 510
Val Tyr Leu Gly Glu Thr Val Gln Pro Glu Val Arg Gly Thr Leu Gly
        515                 520                 525
Leu Leu Pro Thr Ala Phe Gly Asn Ile Gly Ile Leu Leu Cys Phe Val
    530                 535                 540
Ala Gly Ser Phe Met Asn Trp Ser Met Leu Ala Phe Leu Gly Ala Ala
545                 550                 555                 560
Leu Pro Val Pro Phe Leu Ile Leu Met Phe Leu Ile Pro Glu Thr Pro
                565                 570                 575
Arg Trp Phe Val Gly Arg Gly Leu Glu Glu Arg Ala Arg Lys Ala Leu
            580                 585                 590
Lys Trp Leu Arg Gly Lys Glu Ala Asp Val Glu Pro Glu Leu Lys Gly
        595                 600                 605
Leu Met Arg Ser Gln Ala Asp Ala Asp Arg Gln Ala Ser Arg Asn Thr
    610                 615                 620
Met Leu Glu Leu Leu Lys Leu Asn Asn Leu Lys Pro Leu Ser Ile Ser
625                 630                 635                 640
Leu Gly Leu Met Phe Phe Gln Gln Phe Ser Gly Ile Asn Ala Val Ile
                645                 650                 655
Phe Tyr Thr Val Gln Ile Phe Lys Asp Ala Gly Ser Thr Ile Asp Gly
            660                 665                 670
Asn Leu Cys Thr Ile Ile Val Gly Ile Val Asn Phe Leu Ala Thr Phe
        675                 680                 685
Ile Gly Ile Val Leu Ile Asp Arg Ala Gly Arg Lys Ile Leu Leu Tyr
    690                 695                 700
Val Ser Asp Ile Ala Met Val Leu Thr Leu Phe Val Leu Gly Gly Phe
705                 710                 715                 720
Phe Tyr Cys Lys Thr Tyr Gly Pro Asp Val Ser His Leu Gly Trp Leu
                725                 730                 735
Pro Leu Thr Cys Phe Val Ile Tyr Ile Leu Gly Phe Ser Leu Gly Phe
            740                 745                 750
Gly Pro Ile Pro Trp Leu Met Met Gly Glu Ile Leu Pro Ala Lys Ile
        755                 760                 765
Arg Gly Ser Ala Ala Ser Val Ala Thr Ala Phe Asn Trp Phe Cys Thr
    770                 775                 780
Phe Val Val Thr Lys Thr Phe Gln Asp Leu Thr Val Ala Met Gly Ala
785                 790                 795                 800

His Gly Ala Phe Trp Leu Phe Gly Ala Ile Cys Phe Val Gly Leu Phe
                805                 810                 815
Phe Val Ile Ile Tyr Val Pro Glu Thr Gln Gly Lys Thr Leu Glu Asp
            820                 825                 830
Ile Glu Arg Lys Met Met Gly Arg Val Arg Arg Met Ser Ser Val Ala
        835                 840                 845
Asn Ile Lys Pro Leu Ser Phe Asn Met
    850                 855

<210> 5
<211> 6946
<212> DNA
<213> Artificial Sequence

<220>
<223> pSEVA228-setA

<400> 5
ttaattaaac gttcgtaatc aagccacttc ctttttgcat tgacgcaggg tgtcggaagg      60

caactcgccg aacgcgctcc tatagttttc agcgaagcgt cccaaatgta agaagccgta     120

gtctagggct atctcagtta tactacgcac attggcactg ggatcgttca agcaggcgcg     180

gatgctttcg agcttgcggt tgcggatgta gttcttcggc gtggtgccgg cgtgcttctc     240

gaacaaattg tagagcgagc gtggactcat catcgccagc tccgctaacc gctcaaggct     300

gatattccgt ttgagattct cctcaatgaa ttgaacgact cgctcgaaag acgggttacc     360

tttgctgaaa atttcacggc tgacattgct gcccagcatt tcgagcagct tggaagcgat     420

gatccccgca tagtgctctt ggacccgagg catcgacttt gtatgttccg cttcgtcaca     480

aactaacccg agtagattga taaagccatc gagttgctgg agattgtgtc gcgcggcgaa     540

acggataccc tccctcggct tgtgccaatt gttgtcactg cacgcccgat caaggaccac     600

tgagggcaat ttaacgataa atttctcgca atcttctgaa taggtcaggt cggcttggtc     660

atccggattg accttggagc agcaatagtt cgcccggcgc aaaatagtgc tcctggccat     720

ggccacgcca caggcaatgg cctttgagta ttatttgcag atgataacag gtctctaatc     780

caggcgagat taccctcacg ctaccgccgt agctgattcg acacagatcg aggcatccga     840

agattctgtg gtgcagcctg cctgccgggc gcccgccctt gggcaggcga atagagtgcg     900

taccgacata ctggttaaca taatcggaga ctgcataggg ctcggcgtgg acgaagatct     960

gacttttctc gttcaataag caaaaatcca tagttcacgg ttctcttatt ttaatgtggg    1020

ctgcttggtg tgatgtagaa aggcgccaag tcgatgaaaa tgcatctcga cgtgatgcgt    1080

atacgggtta cccccattgc cacgttgcgc catccttttt gcaatcagtg accacttttc    1140

caagcaaaaa taacgccaag cagaacgaag acgttctttt taagaagcga gaacaccaga    1200

agttcgtgct gtcggggcat ggggcgacga attggcggat aaaggggatc tgctggatat    1260

tacggccttt ttaaagaccg taaagaaaaa taagcacaag ttttatccgg cctttattca    1320

cattcttgcc cgcctgatga atgctcatcc gtaattacgt atggcaatga aagacggtga 1380

gctggtgata tgggatagtg ttcacccttg ttacaccgtt ttccatgagc aaactgaaac 1440

gttttcatcg ctctggagtg aataccacga cgatttccgg cagtttctac acatatattc 1500

gcaagatgtg gcgtgttacg gtgaaaacct ggcctatttc cctaaagggt ttattgagaa 1560

tatgtttttc gtctcagcca atccctgggt gagtttcacc agttttgatt taaacgtggc 1620

caatatggac aacttcttcg cccccgtttt caccatgcat gggaattagc ttgatctgac 1680

caacgaccgg tagcggagct atccaacggc ggtataccag gaaaacacac agcaggtaca 1740

tcagaacagt accatgactg aagaacaaat agttttttcc tgatccataa agcagaacgg 1800

cctgctccat gacaaatctg gctccccaac taatgcccca tgcagccagc ataaccagca 1860

taaagtgcag tgtccggttt gatagggata agtccagcct tgcaagaagc ggatacagga 1920

gtgcaaaaaa tggctatctc tagtaaggcc taccccttag gctttatgca acagaaacaa 1980

taataatgga gtcatgacca tgcctaggcc gcggccgcgc gaattcagga ggcttcatat 2040

gatctggata atgacgatgg ctcgccgtat gaacggtgtt tacgcggcat ttatgctggt 2100

cgcttttatg atgggggtgg ccggggcgct acaggctcct acattgagct tatttctgag 2160

tcgtgaggtt ggcgcgcaac ctttctggat cggcctcttt tatacggtga atgctattgc 2220

tgggatcggc gtaagcctct ggttggcaaa acgttctgac agtcagggcg atcggcgaaa 2280

actgattata ttttgctgtt tgatggctat cggcaatgcg ctattgtttg catttaatcg 2340

tcattatctg acgcttatca cctgtggtgt gcttctggca tctctggcca atacggcaat 2400

gccacagtta tttgctctgg cgcgggaata tgcggataac tcggcgcgag aagtggtgat 2460

gtttagctcg gtgatgcgtg cgcagctttc tctggcatgg gttatcggtc caccgttggc 2520

ctttatgctg gcgttgaatt acggctttac ggtgatgttt tcgattgccg ccgggatatt 2580

cacactcagt ctggtattga ttgcatttat gcttccgtct gtggcgcggg tagaactgcc 2640

gtcggaaaat gctttatcaa tgcaaggtgg ctggcaggat agtaacgtac ggatgttatt 2700

tgtcgcctcg acgttaatgt ggacctgcaa caccatgtac attattgata tgccgttgtg 2760

gatcagtagc gagttaggat tgccagacaa actggcgggt ttcctgatgg ggacggcagc 2820

tggactggaa ataccagcaa tgattctggc tggctactat gtcaaacgtt atggtaagcg 2880

gcgaatgatg gtcatagcag tggcggcagg agtactgttt tacaccggat tgattttctt 2940

taatagccgt atggcgttga tgacgctgca actttttaac gctgtattta tcggcattgt 3000

tgcgggtatt gggatgctat ggtttcagga tttaatgcct ggaagagcgg gggcagctac 3060

caccttattt actaacagta tttctaccgg ggtaattctg gctggcgtta ttcagggagc 3120

aattgcacaa agttgggggc actttgctgt ctactgggta attgcggtta tttctgttgt 3180

cgcattattt ttaaccgcaa aggttaaaga cgtttgactg caggcatgca agcttgcggc 3240

cgcgtcgtga ctgggaaaac cctggcgact agtcttggac tcctgttgat agatccagta 3300

atgacctcag aactccatct ggatttgttc agaacgctcg gttgccgccg ggcgtttttt 3360

attggtgaga atccaggggt ccccaataat tacgatttaa atttgtgtct caaaatctct 3420

gatgttacat tgcacaagat aaaaatatat catcatgaac aataaaactg tctgcttaca 3480

taaacagtaa tacaaggggt gttatgagcc atattcagcg tgaaacgagc tgtagccgtc 3540

cgcgtctgaa cagcaacatg gatgcggatc tgtatggcta taaatgggcg cgtgataacg 3600

tgggtcagag cggcgcgacc atttatcgtc tgtatggcaa accggatgcg ccggaactgt 3660

ttctgaaaca tggcaaaggc agcgtggcga acgatgtgac cgatgaaatg gtgcgtctga 3720

actggctgac cgaatttatg ccgctgccga ccattaaaca ttttattcgc accccggatg 3780

atgcgtggct gctgaccacc gcgattccgg gcaaaaccgc gtttcaggtg ctggaagaat 3840

atccggatag cggcgaaaac attgtggatg cgctggccgt gtttctgcgt cgtctgcata 3900

gcattccggt gtgcaactgc ccgtttaaca gcgatcgtgt gtttcgtctg gcccaggcgc 3960

agagccgtat gaacaacggc ctggtggatg cgagcgattt tgatgatgaa cgtaacggct 4020

ggccggtgga acaggtgtgg aaagaaatgc ataaactgct gccgtttagc ccggatagcg 4080

tggtgaccca cggcgatttt agcctggata acctgatttt cgatgaaggc aaactgattg 4140

gctgcattga tgtgggccgt gtgggcattg cggatcgtta tcaggatctg gccattctgt 4200

ggaactgcct gggcgaattt agcccgagcc tgcaaaaacg tctgtttcag aaatatggca 4260

ttgataatcc ggatatgaac aaactgcaat ttcatctgat gctggatgaa tttttctaat 4320

aattaattgg accgcggtcc gcgcgttgtc cttttccgct gcataaccct gcttcggggt 4380

cattatagcg attttttcgg tatatccatc ctttttcgca cgatatacag gattttgcca 4440

aagggttcgt gtagactttc cttggtgtat ccaacggcgt cagccgggca ggataggtga 4500

agtaggccca cccgcgagcg ggtgttcctt cttcactgtc ccttattcgc acctggcggt 4560

gctcaacggg aatcctgctc tgcgaggctg gccgtaggcc ggccgcgatg caggtggctg 4620

ctgaaccccc agccggaact gaccccacaa ggccctagcg tttgcaatgc accaggtcat 4680

cattgaccca ggcgtgttcc accaggccgc tgcctcgcaa ctcttcgcag gcttcgccga 4740

cctgctcgcg ccacttcttc acgcgggtgg aatccgatcc gcacatgagg cggaaggttt 4800

ccagcttgag cgggtacggc tcccggtgcg agctgaaata gtcgaacatc cgtcgggccg 4860

tcggcgacag cttgcggtac ttctcccata tgaatttcgt gtagtggtcg ccagcaaaca 4920

gcacgacgat ttcctcgtcg atcaggacct ggcaacggga cgttttcttg ccacggtcca 4980

ggacgcggaa gcggtgcagc agcgacaccg attccaggtg cccaacgcgg tcggacgtga 5040

agcccatcgc cgtcgcctgt aggcgcgaca ggcattcctc ggccttcgtg taataccggc 5100

cattgatcga ccagcccagg tcctggcaaa gctcgtagaa cgtgaaggtg atcggctcgc 5160

cgataggggt gcgcttcgcg tactccaaca cctgctgcca caccagttcg tcatcgtcgg 5220

cccgcagctc gacgccggtg taggtgatct tcacgtcctt gttgacgtgg aaaatgacct 5280

tgttttgcag cgcctcgcgc gggattttct tgttgcgcgt ggtgaacagg gcagagcggg 5340

ccgtgtcgtt tggcatcgct cgcatcgtgt ccggccacgg cgcaatatcg aacaaggaaa 5400

gctgcatttc cttgatctgc tgcttcgtgt gtttcagcaa cgcggcctgc ttggcttcgc 5460

tgacctgttt tgccaggtcc tcgccggcgg tttttcgctt cttggtcgtc atagttcctc 5520

gcgtgtcgat ggtcatcgac ttcgccaaac ctgccgcctc ctgttcgaga cgacgcgaac 5580

gctccacggc ggccgatggc gcgggcaggg cagggggagc cagttgcacg ctgtcgcgct 5640

cgatcttggc cgtagcttgc tggactatcg agccgacgga ctggaaggtt tcgcggggcg 5700

cacgcatgac ggtgcggctt gcgatggttt cggcatcctc ggcggaaaac cccgcgtcga 5760

tcagttcttg cctgtatgcc ttccggtcaa acgtccgatt cattcaccct ccttgcggga 5820

ttgccccgga attaattccc cggatcgatc cgtcgatctt gatcccctgc gccatcagat 5880

ccttggcggc aagaaagcca tccagtttac tttgcagggc ttcccaacct taccagaggg 5940

cgccccagct ggcaattccg gttcgcttgc tgtccataaa accgcccagt ctagctatcg 6000

ccatgtaagc ccactgcaag ctacctgctt tctctttgcg cttgcgtttt cccttgtcca 6060

gatagcccag tagctgacat tcatccgggg tcagcaccgt ttctgcggac tggctttcta 6120

cgtggctgcc atttttgggg tgaggccgtt cgcggccgag gggcgcagcc cctgggggga 6180

tgggaggccc gcgttagcgg gccgggaggg ttcgagaagg gggggcaccc cccttcggcg 6240

tgcgcggtca cgcgcacagg gcgcagccct ggttaaaaac aaggtttata aatattggtt 6300

taaaagcagg ttaaaagaca ggttagcggt ggccgaaaaa cgggcggaaa cccttgcaaa 6360

tgctggattt tctgcctgtg gacagcccct caaatgtcaa taggtgcgcc cctcatctgt 6420

cagcactctg cccctcaagt gtcaaggatc gcgcccctca tctgtcagta gtcgcgcccc 6480

tcaagtgtca ataccgcagg gcacttatcc ccaggcttgt ccacatcatc tgtgggaaac 6540

tcgcgtaaaa tcaggcgttt tcgccgattt gcgaggctgg ccagctccac gtcgccggcc 6600

gaaatcgagc ctgcccctca tctgtcaacg ccgcgccggg tgagtcggcc cctcaagtgt 6660

caacgtccgc ccctcatctg tcagtgaggg ccaagttttc cgcgaggtat ccacaacgcc 6720

ggcggcccta catggctctg ctgtagtgag tgggttgcgc tccggcagcg gtcctgatcc 6780

cccgcagaaa aaaaggatct caagaagatc ctttgatctt ttctacggcg cgcccagctg 6840

tctagggcgg cggatttgtc ctactcagga gagcgttcac cgacaaacaa cagataaaac 6900

gaaaggccca gtctttcgac tgagcctttc gttttatttg atgcct 6946

<210> 6
<211> 8786
<212> DNA
<213> Artificial Sequence

<220>
<223> pSEVA228-galP-treF

<400> 6
ttaattaaac gttcgtaatc aagccacttc ctttttgcat tgacgcaggg tgtcggaagg 60

caactcgccg aacgcgctcc tatagttttc agcgaagcgt cccaaatgta agaagccgta 120

gtctagggct atctcagtta tactacgcac attggcactg ggatcgttca agcaggcgcg 180

gatgctttcg agcttgcggt tgcggatgta gttcttcggc gtggtgccgg cgtgcttctc 240

gaacaaattg tagagcgagc gtggactcat catcgccagc tccgctaacc gctcaaggct 300

gatattccgt ttgagattct cctcaatgaa ttgaacgact cgctcgaaag acgggttacc 360

tttgctgaaa atttcacggc tgacattgct gcccagcatt tcgagcagct tggaagcgat 420

gatccccgca tagtgctctt ggacccgagg catcgacttt gtatgttccg cttcgtcaca 480

aactaacccg agtagattga taaagccatc gagttgctgg agattgtgtc gcgcggcgaa 540

acggataccc tccctcggct tgtgccaatt gttgtcactg cacgcccgat caaggaccac 600

tgagggcaat ttaacgataa atttctcgca atcttctgaa taggtcaggt cggcttggtc 660

atccggattg agcagcaata gttcgcccgg cgcaaaatag tgctcctggc catggccacg 720

ccacaggcaa tggcctttga gtattatttg cagatgataa caggtctcta atccaggcga 780

gattaccctc acgctaccgc cgtagctgat tcgacacaga tcgaggcatc cgaagattct 840

gtggtgcagc ctgcctgccg ggcgcccgcc cttgggcagg cgaatagagt gcgtaccgac 900

atactggtta acataatcgg agactgcata gggctcggcg tggacgaaga tctgactttt 960

ctcgttcaat aagcaaaaat ccatagttca cggttctctt attttaatgt gggctgcttg 1020

gtgtgatgta gaaaggcgcc aagtcgatga aaatgcatct cgacgtgatg cgtatacggg 1080

ttacccccat tgccacgttg cgccatcctt tttgcaatca gtgaccactt ttccaagcaa 1140

aaataacgcc aagcagaacg aagacgttct ttttaagaag cgagaacacc agaagttcgt 1200

gctgtcgggg catggggcga cgaattggcg gataaagggg atctgctgga tattacggcc 1260

tttttaaaga ccgtaaagaa aaataagcac aagttttatc cggcctttat tcacattctt 1320

gcccgcctga tgaatgctca tccgtaatta cgtatggcaa tgaaagacgg tgagctggtg 1380

atatgggata gtgttcaccc ttgttacacc gttttccatg agcaaactga aacgttttca 1440

tcgctctgga gtgaatacca cgacgatttc cggcagtttc tacacatata ttcgcaagat 1500

gtggcgtgtt acggtgaaaa cctggcctat ttccctaaag ggtttattga gaatatgttt 1560

ttcgtctcag ccaatccctg ggtgagtttc accagttttg atttaaacgt ggccaatatg 1620

gacaacttct tcgcccccgt tttcaccatg catgggaatt agcttgatct gaccaacgac 1680

cggtagcgga gctatccaac ggcggtatac caggaaaaca cacagcaggt acatcagaac 1740

agtaccatga ctgaagaaca aatagttttt tcctgatcca taaagcagaa cggcctgctc 1800

catgacaaat ctggctcccc aactaatgcc ccatgcagcc agcataacca gcataaagtg 1860

cagtgtccgg tttgataggg ataagtccag ccttgcaaga agcggataca ggagtgcaaa 1920

aaatggctat ctctagtaag gcctacccct taggctttat gcaacagaaa caataataat 1980

ggagtcatga ccatgcctag gccgcggccg cgcgaattcg agctctaacc atattggagg 2040

gcatcatgcc tgacgctaaa aaacaggggc ggtcaaacaa ggcaatgacg tttttcgtct 2100

gcttccttgc cgctctggcg ggattactct ttggcctgga tatcggtgta attgctggcg 2160

cactgccgtt tattgcagat gaattccaga ttacttcgca cacgcaagaa tgggtcgtaa 2220

gctccatgat gttcggtgcg gcagtcggtg cggtgggcag cggctggctc tcctttaaac 2280

tcgggcgcaa aaagagcctg atgatcggcg caattttgtt tgttgccggt tcgctgttct 2340

ctgcggctgc gccaaacgtt gaagtactga ttctttcccg cgttctactg gggctggcgg 2400

tgggtgtggc ctcttatacc gcaccgctgt acctctctga aattgcgccg gaaaaaattc 2460

gtggcagtat gatctcgatg tatcagttga tgatcactat cgggatcctc ggtgcttatc 2520

tttctgatac cgccttcagc tacaccggtg catggcgctg gatgctgggt gtgattatca 2580

tcccggcaat tttgctgctg attggtgtct tcttcctgcc agacagccca cgttggtttg 2640

ccgccaaacg ccgttttgtt gatgccgaac gcgtgctgct acgcctgcgt gacaccagcg 2700

cggaagcgaa acgcgaactg gatgaaatcc gtgaaagttt gcaggttaaa cagagtggct 2760

gggcgctgtt taaagagaac agcaacttcc gccgcgcggt gttccttggc gtactgttgc 2820

aggtaatgca gcaattcacc gggatgaacg tcatcatgta ttacgcgccg aaaatcttcg 2880

aactggcggg ttataccaac actaccgagc aaatgtgggg gaccgtgatt gtcggcctga 2940

ccaacgtact tgccaccttt atcgcaatcg gccttgttga ccgctgggga cgtaaaccaa 3000

cgctaacgct gggcttcctg gcgatggctg ctggcatggg cgtactcggt acaatgatgc 3060

atatcggtat tcactctccg tcggcgcagt atttcgccat cgccatgctg ctgatgttta 3120

ttgtcggttt tgccatgagt gccggtccgc tgatttgggt actgtgctcc gaaattcagc 3180

cgctgaaagg ccgcgatttt ggcatcacct gctccactgc caccaactgg attgccaaca 3240

tgatcgttgg cgcaacgttc ctgaccatgc tcaacacgct gggtaacgcc aacaccttct 3300

gggtgtatgc ggctctgaac gtactgttta tcctgctgac attgtggctg gtaccggaaa 3360

ccaaacacgt ttcgctggaa catattgaac gtaatctgat gaaaggtcgt aaactgcgcg 3420

aaataggcgc tcacgattaa aagcttagga ggcttcatat gctcaatcag aaaattcaaa 3480

accctaatcc agacgaactg atgatcgaag tcgatctctg ctatgagctg gacccgtatg 3540

aattaaaact ggatgagatg atcgaggcag aaccggaacc cgagatgatt gaagggctgc 3600

ctgcctctga tgcgctgacg cctgccgatc gctatctcga actgttcgag catgttcagt 3660

cggcgaaaat tttccccgac agtaaaacct ttcccgactg cgcacctaaa atggacccgc 3720

tggatatctt aatccgctac cgtaaagtgc gccgtcatcg tgattttgac ttgcgcaagt 3780

ttgttgaaaa ccacttctgg ctgccggagg tctactccag cgagtatgta tcggacccgc 3840

aaaattccct gaaagagcat atcgaccagc tgtggccggt gctaacccgc gaaccacagg 3900

atcacattcc gtggtcttct ctgctggcgc tgccgcagtc atatattgtc ccgggcggcc 3960

gttttagcga aacctactat tgggattcct atttcaccat gctggggctg gcggaaagtg 4020

gtcgggaaga tttgctgaaa tgcatggccg ataacttcgc ctggatgatc gaaaactacg 4080

gtcacatccc caacggcaac cgcacctatt atttgagccg ctcgcaacca ccggtttttg 4140

cgctgatggt ggagttgttt gaagaagatg gtgtacgcgg tgcgcgccgc tatctcgacc 4200

accttaaaat ggaatatgcc ttctggatgg acggtgcaga atcgttaatc cctaatcagg 4260

cctatcgcca tgttgtgcgg atgccggacg gatcgctgct caaccgttac tgggacgatc 4320

gcgacacgcc gcgtgacgaa tcctggcttg aggacgttga aaccgcgaaa cattctggtc 4380

gcccgcccaa cgaggtgtac cgcgatttac gcgcgggggc ggcctccggt tgggattact 4440

cttcccgttg gctgcgtgat actggtcgtc tggcgagcat tcgtaccacc cagttcatcc 4500

ccatcgatct gaatgccttc ctgtttaaac tggagagcgc catcgccaac atctcggcgc 4560

tgaaaggcga gaaagagaca gaagcactgt tccgccagaa agccagtgcc cgtcgcgatg 4620

cggtaaaccg ttacctctgg gatgatgaaa acggcatcta ccgcgattac gactggcgac 4680

gcgaacaact ggcgctgttt tccgctgccg ccattgtgcc actctatgtc ggtatggcga 4740

accatgaaca ggccgatcgt ctggcaaacg ccgtgcgcag tcggttactg acacctggcg 4800

ggattctggc aagcgagtac gaaaccggtg aacagtggga taaacccaac ggctgggcac 4860

cgttacaatg gatggcgatt cagggattta aaatgtacgg cgatgacctt ctgggtgatg 4920

aaatcgcgcg aagctggctg aagacggtga atcagttcta tctggaacag cacaaactga 4980

tcgaaaaata ccatattgcc gatggtgttc cccgcgaagg cggcggtggc gagtatccgt 5040

tgcaggatgg gtttggctgg actaacggtg tggtacgccg tttaattggt ttgtacggcg 5100

aaccataact agtcttggac tcctgttgat agatccagta atgacctcag aactccatct 5160

ggatttgttc agaacgctcg gttgccgccg ggcgtttttt attggtgaga atccaggggt 5220

ccccaataat tacgatttaa atttgtgtct caaaatctct gatgttacat tgcacaagat 5280

aaaaatatat catcatgaac aataaaactg tctgcttaca taaacagtaa tacaaggggt 5340

gttatgagcc atattcagcg tgaaacgagc tgtagccgtc cgcgtctgaa cagcaacatg 5400

gatgcggatc tgtatggcta taaatgggcg cgtgataacg tgggtcagag cggcgcgacc 5460

atttatcgtc tgtatggcaa accggatgcg ccggaactgt ttctgaaaca tggcaaaggc 5520

agcgtggcga acgatgtgac cgatgaaatg gtgcgtctga actggctgac cgaatttatg 5580

ccgctgccga ccattaaaca ttttattcgc accccggatg atgcgtggct gctgaccacc 5640

gcgattccgg gcaaaaccgc gtttcaggtg ctggaagaat atccggatag cggcgaaaac 5700

attgtggatg cgctggccgt gtttctgcgt cgtctgcata gcattccggt gtgcaactgc 5760

ccgtttaaca gcgatcgtgt gtttcgtctg gcccaggcgc agagccgtat gaacaacggc 5820

ctggtggatg cgagcgattt tgatgatgaa cgtaacggct ggccggtgga acaggtgtgg 5880

aaagaaatgc ataaactgct gccgtttagc ccggatagcg tggtgaccca cggcgatttt 5940

agcctggata acctgatttt cgatgaaggc aaactgattg gctgcattga tgtgggccgt 6000

gtgggcattg cggatcgtta tcaggatctg gccattctgt ggaactgcct gggcgaattt 6060

agcccgagcc tgcaaaaacg tctgtttcag aaatatggca ttgataatcc ggatatgaac 6120

aaactgcaat ttcatctgat gctggatgaa tttttctaat aattaattgg accgcggtcc 6180

gcgcgttgtc cttttccgct gcataaccct gcttcggggt cattatagcg attttttcgg 6240

tatatccatc ctttttcgca cgatatacag gattttgcca aagggttcgt gtagactttc 6300

cttggtgtat ccaacggcgt cagccgggca ggataggtga agtaggccca cccgcgagcg 6360

ggtgttcctt cttcactgtc ccttattcgc acctggcggt gctcaacggg aatcctgctc 6420

tgcgaggctg gccgtaggcc ggccgcgatg caggtggctg ctgaaccccc agccggaact 6480

gaccccacaa ggccctagcg tttgcaatgc accaggtcat cattgaccca ggcgtgttcc 6540

accaggccgc tgcctcgcaa ctcttcgcag gcttcgccga cctgctcgcg ccacttcttc 6600

acgcgggtgg aatccgatcc gcacatgagg cggaaggttt ccagcttgag cgggtacggc 6660

tcccggtgcg agctgaaata gtcgaacatc cgtcgggccg tcggcgacag cttgcggtac 6720

ttctcccata tgaatttcgt gtagtggtcg ccagcaaaca gcacgacgat ttcctcgtcg 6780

atcaggacct ggcaacggga cgttttcttg ccacggtcca ggacgcggaa gcggtgcagc 6840

agcgacaccg attccaggtg cccaacgcgg tcggacgtga agcccatcgc cgtcgcctgt 6900

aggcgcgaca ggcattcctc ggccttcgtg taataccggc cattgatcga ccagcccagg 6960

tcctggcaaa gctcgtagaa cgtgaaggtg atcggctcgc cgataggggt gcgcttcgcg 7020

tactccaaca cctgctgcca caccagttcg tcatcgtcgg cccgcagctc gacgccggtg 7080

taggtgatct tcacgtcctt gttgacgtgg aaaatgacct tgttttgcag cgcctcgcgc 7140

gggattttct tgttgcgcgt ggtgaacagg gcagagcggg ccgtgtcgtt tggcatcgct 7200

cgcatcgtgt ccggccacgg cgcaatatcg aacaaggaaa gctgcatttc cttgatctgc 7260

tgcttcgtgt gtttcagcaa cgcggcctgc ttggcttcgc tgacctgttt tgccaggtcc 7320

tcgccggcgg tttttcgctt cttggtcgtc atagttcctc gcgtgtcgat ggtcatcgac 7380

ttcgccaaac ctgccgcctc ctgttcgaga cgacgcgaac gctccacggc ggccgatggc 7440

gcgggcaggg cagggggagc cagttgcacg ctgtcgcgct cgatcttggc cgtagcttgc 7500

tggactatcg agccgacgga ctggaaggtt tcgcggggcg cacgcatgac ggtgcggctt 7560

gcgatggttt cggcatcctc ggcggaaaac cccgcgtcga tcagttcttg cctgtatgcc 7620

ttccggtcaa acgtccgatt cattcaccct ccttgcggga ttgccccgga attaattccc 7680

cggatcgatc cgtcgatctt gatcccctgc gccatcagat ccttggcggc aagaaagcca 7740

tccagtttac tttgcagggc ttcccaacct taccagaggg cgccccagct ggcaattccg 7800

gttcgcttgc tgtccataaa accgcccagt ctagctatcg ccatgtaagc ccactgcaag 7860

ctacctgctt tctctttgcg cttgcgtttt cccttgtcca gatagcccag tagctgacat 7920

tcatccgggg tcagcaccgt ttctgcggac tggctttcta cgtggctgcc atttttgggg 7980

tgaggccgtt cgcggccgag gggcgcagcc cctgggggga tgggaggccc gcgttagcgg 8040

gccgggaggg ttcgagaagg gggggcaccc cccttcggcg tgcgcggtca cgcgcacagg 8100

gcgcagccct ggttaaaaac aaggtttata aatattggtt taaaagcagg ttaaaagaca 8160

ggttagcggt ggccgaaaaa cgggcggaaa cccttgcaaa tgctggattt tctgcctgtg 8220

gacagcccct caaatgtcaa taggtgcgcc cctcatctgt cagcactctg cccctcaagt 8280

gtcaaggatc gcgcccctca tctgtcagta gtcgcgcccc tcaagtgtca ataccgcagg 8340

gcacttatcc ccaggcttgt ccacatcatc tgtgggaaac tcgcgtaaaa tcaggcgttt 8400

tcgccgattt gcgaggctgg ccagctccac gtcgccggcc gaaatcgagc ctgcccctca 8460

tctgtcaacg ccgcgccggg tgagtcggcc cctcaagtgt caacgtccgc ccctcatctg 8520

tcagtgaggg ccaagttttc cgcgaggtat ccacaacgcc ggcggcccta catggctctg 8580

ctgtagtgag tgggttgcgc tccggcagcg gtcctgatcc cccgcagaaa aaaaggatct 8640

caagaagatc ctttgatctt ttctacggcg cgcccagctg tctagggcgg cggatttgtc 8700

ctactcagga gagcgttcac cgacaaacaa cagataaaac gaaaggccca gtctttcgac 8760

tgagcctttc gttttatttg atgcct 8786